Europäisches Patentamt
European Patent Office
Office européen des brevets

(19)

(11) Veröffentlichungsnummer: **0 026 409 A1**

# (12) EUROPÄISCHE PATENTANMELDUNG

(21) Anmeldenummer: 80105635.9

(22) Anmeldetag: 19.09.80

(51) Int. Cl.³: **A 61 K 37/16** //(A61K37/16, 31/42), (A61K37/16, 31/43), (A61K37/16, 31/545)

(30) Priorität: 28.09.79 GB 7933697

(43) Veröffentlichungstag der Anmeldung: 08.04.81 Patentblatt 81/14

(84) Benannte Vertragsstaaten: AT BE CH DE FR GB IT LI LU NL SE

(71) Anmelder: F. HOFFMANN-LA ROCHE & CO. Aktiengesellschaft

CH-4002 Basel(CH)

(72) Erfinder: Atherton, Frank Ratcliffe
148 Park Way
Welwyn Garden City, Herts.(GB)

(72) Erfinder: Hall, Michael John
15 Cannonsfield Road
Welwyn, Herts.(GB)

(72) Erfinder: Hassall, Cedric, Herbert
23 The Ryde
Hatfield, Herts.(GB)

(72) Erfinder: Lambert, Robert Wilson
6 Grange Hill
Welwyn, Herts.(GB)

(72) Erfinder: Ringrose, Peter Stuart
10 Geneegasse
A-1130 Wien(AT)

(74) Vertreter: Lederer, Franz, Dr. et al,
Patentanwälte Dr. Franz Lederer Reiner F. Meyer
Lucile-Grahn-Strasse 22
D-8000 München 80(DE)

## (54) Wirkstoffkombinationen, deren Verwendung und pharmazeutische Präparate sowie deren Herstellung.

(57) Wirkstoffkombinationen mit antibiotischen Eigenschaften, dadurch gekennzeichnet, dass sie ein Peptid-derivat der allgemeinen Formel

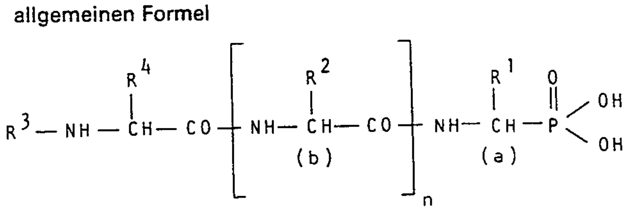

(1)

oder ein physiologisch verträgliches Salz einer solchen Verbindung und ein Antibiotikum enthalten, deren Verwendung und pharmazeutische Präparate sowie deren Herstellung.

worin $R^1$ Wasserstoff, Methyl, Hydroxymethyl, eine Mono-, Di- oder Trihalogenmethylgruppe ist;

$R^2$ einen für eine normalerweise in Proteinen vorkommende α-Aminosäure charakteristischen Rest oder einen Niederalkyl- oder Hydroxyniederalkylrest, der charakterisch ist für eine in Proteinen normalerweise nicht vorkommende α-Aminosäure, darstellt;

$R^3$ Niederalkyl, Nieder-cycloalkyl, Nieder-alkenyl, Aryl oder Aryl-niederalkyl ist;

$R^4$ Wasserstoff oder Niederalkyl bedeutet;

n 2 oder 3 bedeutet und

die Konfigurationen an den C-Atomen (a) und (b)

R (wenn $R^1 \neq$ H) bzw. L sind,

EP 0 026 409 A1

0026409

F.Hoffmann-La Roche & Co. Aktiengesellschaft,Basel,Schweiz

19. Sep. 1980
RAN 4105/54

# Wirkstoffkombinationen, deren Verwendung und pharmazeutische Präparate sowie deren Herstellung.

Peptidderivate der allgemeinen Formel

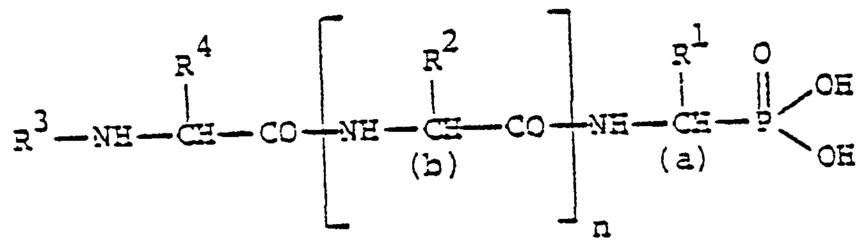

(I)

worin $R^1$ Wasserstoff, Methyl, Hydroxymethyl, eine Mono-, Di- oder Trihalogenmethylgruppe ist;

$R^2$ einen für eine normalerweise in Proteinen vorkommende α-Aminosäure charakteristischen Rest oder einen Niederalkyl- oder Hydroxy-niederalkylrest, der charakterisch ist für eine in Proteinen normalerweise nicht vorkommende α-Aminosäure, darstellt;

$R^3$ Niederalkyl, Nieder-cycloalkyl, Nieder-alkenyl, Aryl oder Aryl-niederalkyl ist;

$R^4$ Wasserstoff oder Niederalkyl bedeutet;

n 2 oder 3 bedeutet und

Mez/ 1.9.80

die Konfigurationen an den C-Atomen (a) und (b) R (wenn $R^1 \neq H$) bzw. L sind,

und deren physiologisch verträglichen Salze sind bekannt. Diese Verbindungen besitzen antibakterielle Aktivität.

Es wurde nun gefunden, dass die Peptidderivate der Formel I und ihre Salze die Aktivität von Antibiotika potenzieren.

Die vorliegende Erfindung betrifft daher Wirkstoffkombinationen, die ein Peptidderivat der Formel I oder ein physiologisch verträgliches Salz davon enthalten, sowie deren Herstellung und Verwendung.

Die Bezeichnung "Niederalkyl" soll im vorliegenden Zusammenhang eine gerad- oder verzweigtkettige Alkylgruppe mit vorzugsweise bis zu 8 C-Atomen bedeuten, z.B. Methyl, Ethyl, Propyl, Isopropyl, Butyl, tert.Butyl, Pentyl, Hexyl. Beispiele von Hydroxyniederalkylgruppen sind 2-Hydroxyethyl, 3-Hydroxypropyl, 4-Hydroxybutyl. Die Bezeichnung "Niedercycloalkyl" bedeutet eine Cycloalkylgruppe mit vorzugsweise 3-6 C-Atomen, z.B. Cyclopropyl, Cyclobutyl. Die Bezeichnung "Niederalkenyl" bedeutet eine gerad- oder verzweigtkettige Alkenylgruppe mit vorzugsweise 2-8 C-Atomen, z.B. Allyl, Butenyl. Beispiele von Arylgruppen sind Phenyl, Tolyl und Beispiele von Arylniederalkylgruppen sind Benzyl, Phenethyl. Die Bezeichnung "Halogen" bedeutet Fluor, Chlor, Brom oder Jod. Beispiele der oben erwähnten Halogenmethylgruppen sind Chlormethyl, Dichlormethyl, Trifluormethyl. Die Bezeichnung "charakteristischer Reste einer normalerweise in Proteinen vorkommenden α-Aminosäure" bedeutet den Rest R einer natürlichen α-Aminosäure der allgemeinen Formel

$$H_2N-\overset{\overset{R}{|}}{C}H-COOH$$

wie sie normalerweise in Proteinen vorkommen. So bedeutet R, falls die α-Aminosäure Glycin ist, Wasserstoff. Wenn die α-Aminosäure Alanin ist, bedeutet R Methyl. Im Methionin bedeutet R 2-Methylthioethyl, im Serin Hydroxymethyl und im Tyrosin p-Hydroxybenzyl. R kann auch zusammen mit dem Stickstoff der Aminogruppe zum Ring geschlossen sein, wie im Prolin.

Wenn $R^1$ in der Formel I nicht Wasserstoff darstellt, dann soll die Konfiguration am mit (a) bezeichneten C-Atom (R) sein, d.h. die Konfiguration soll so sein, wie sie beim Ersatz der Carboxylgruppe einer natürlichen α-Aminosäure durch eine $PO_3H_2$-Gruppe erhalten würde.

Die Reste $R^2$ in Formel I können im Rahmen der angegebenen Definitionen gleich oder verschieden voneinander sein.

Bevorzugte Peptidderivate der Formel I sind einerseits solche, in denen $R^1$ Wasserstoff oder Methyl ist, andererseits solche, in denen die Reste $R^2$ einen für eine normalerweise in Proteinen vorkommenden α-Aminosäure charakteristischen Rest oder einen Niederalkylrest darstellen, der charakteristisch ist für eine normalerweise in Proteinen nicht vorkommende α-Aminosäure. Schliesslich sind solche Peptidderivate der Formel I bevorzugt, in denen $R^3$ Niederalkyl, insbesondere Methyl ist.

Beispiele von Peptidderivaten der Formel I sind:

(1R)-1-(N-Sarcosyl-glycyl-L-alanylamino)-ethylphosphonsäure,

(1R)-1-(N-Sarcosyl-L-alanyl-L-alanylamino)-ethylphosphonsäure,

(1R)-1-(N-Sarcosyl-L-methionyl-L-alanylamino)-ethylphosphonsäure,

(1R)-1-(N-Sarcosyl-L-histidyl-L-alanylamino)-ethylphosphonsäure,

(1R)-1-(N-Sarcosyl-L-seryl-L-alanylamino)-ethylphosphonsäure,

(1R)-1-(N-Sarcosyl-L-tyrosyl-L-alanylamino)-ethylphosphonsäure,

(1R)-1-(N-Sarcosyl-L-arginyl-L-alanylamino)-ethylphosphonsäure,

(1R)-1-(N-Sarcosyl-L-alanyl-L-arginylamino)-ethylphosphonsäure,

(1R)-1-(N-Sarcosyl-L-alanyl-L-serylamino)-ethylphosphonsäure,

(1R)-1-N-Sarcosyl-L-alanyl-L-histidylamino)-ethylphosphonsäure,

(N-Sarcosyl-L-alanyl-L-alanylamino)-methylphosphonsäure,

(1R)-1-(N-Sarcosyl-L-norvalyl-L-alanylamino)-ethylphosphonsäure,

(1R)-1-(N-Sarcosyl-L-alanyl-L-norvalylamino)-ethylphosphonsäure,

(1R)-1-(N-Sarcosyl-L-norvalyl-L-norvalylamino)-ethylphosphonsäure,

(1R)-1-(N-Sarcosyl-L-arginyl-L-arginylamino)-ethylphosphonsäure,

(1R)-1-(N-Sarcosyl-L-norvalyl-L-arginylamino)-ethylphosphonsäure,

(1R)-1-(N-Sarcosyl-L-glycyl-L-norvalylamino-ethylphosphonsäure,

(1R)-1-(N-Sarcosyl-L-arginyl-L-norvalylamino)-ethylphosphonsäure,

(1R)-1-(N-Sarcosyl-L-valyl-L-norvalylamino)-ethylphosphonsäure,

(1R)-1-(N-Sarcosyl-glycyl-L-norvalyl-L-norvalylamino)-ethylphosphonsäure,

(N-Sarcosyl-L-norvalyl-L-norvalylamino)-methylphosphonsäure,

(1R)-1-(N-Methyl-L-norvalyl-L-norvalyl-L-norvalylamino)-ethylphosphonsäure,

(1R)-1-(N-Ethyl-glycyl-L-alanyl-L-alanylamino)-ethylphosphonsäure,

(1R)-1-(N-n-Propyl)-glycyl-L-alanyl-L-alanylamino)-ethylphosphonsäure,

(1R)-1-(N-Allyl-glycyl-L-alanyl-L-alanylamino)-ethylphosphonsäure,

(1R)-1-(N-n-Hexyl)-glycyl-L-alanyl-L-alanylamino)-ethylphosphonsäure,

(1R)-1-(N-Cyclopropyl-glycyl-L-alanyl-L-alanylamino)-ethylphosphonsäure,

(1R)-1-(N-tert.Butyl-glycyl-L-alanyl-L-alanylamino)-ethylphosphonsäure,

(1R)-1-(N-Benzyl-glycyl-L-alanyl-L-alanylamino)-ethylphosphonsäure,

(1R)-1-(N-Phenyl-glycyl-L-alanyl-L-alanylamino)-ethylphosphonsäure,

(1R)-1-(N-Methyl-L-alanyl-L-alanyl-L-alanylamino)-ethylphosphonsäure,

(1R)-1-(N-Methyl-L-valyl-L-valyl-L-norvalylamino)-ethylphosphonsäure,

(1R)-1-(N-Methyl-L-leucyl-L-norvalyl-L-norvalylamino)-ethylphosphonsäure,

(1R)-1-(N-Sarcosyl-L-valyl-L-valyl-L-norvalylamino)-ethylphosphonsäure, und

(1R)-1-(N-Methyl-L-valyl-L-norvalyl-L-norvalylamino)-ethylphosphonsäure.

Von dem vorstehend genannten Peptidderivaten ist (1R)-1-(N-Sarcosyl-L-norvalyl-L-norvalylamino)-ethylphosphonsäure bevorzugt.

Physiologisch verträgliche Salze bilden die Peptidderivate der Formel I mit physiologisch verträglichen starken organischen und anorganischen Säuren (z.B. HCl, HBr, $H_2SO_4$, Methansulfonsäure, p-Toluolsulfonsäure) und Basen (z.B. NaOH, KOH).

Die Antibiotika-Komponente der erfindungsgemässen Wirkstoffkombinationen ist vorzugsweise ein β-Lactam-Antibiotikum, wie ein Penicillin, ein Cephalosporin oder

ein monocyclisches β-Lactam-Antibiotikum. Als Penicilline kommen dabei insbesondere Ampicillin, Carbenicillin, Penicillin G, Sulbenicillin, Mecillinam, Pivmecillinam, Phenethicillin, Methicillin, Propicillin, Ticarcillin, Amoxycillin und Piperacillin in Frage; als Cephalosporine insbesondere Cephalexin, Cephazolin, Cefoxitin, Cephradin, Cefsulodin, Cephamandol, Cephaloridin, Cephaloglycin, Cefatrizin, Cefacetril, Cefuroxim, Cefaclor, Cefotaxim und Cefazedone, wobei Cephalexin bevorzugt ist. Ferner kommen D-Cycloserin, Rifampicin, Phosphonomycin, Gentamycin, Vancomycin und Kanamycin als Antibiotika in Betracht.

Das Gewichtsverhältnis einer Verbindung I oder ihrer Salze zum Antibiotikum kann in den erfindungsgemässen Kombinationen innerhalb weiter Grenzen variieren. Im allgemeinen liegt es bei 1:100 bis 100:1, vorzugsweise bei 1:64 bis 64:1. Besonders bevorzugt ist ein Verhältnis von 1:16 bis 16:1.

Die Wirkstoffkombinationen der vorliegenden Anmeldung werden dadurch hergestellt, dass man eine Verbindung der Formel I oder ein physiologisch verträgliches Salz davon und ein Antibiotikum in den oben genannten Gewichtsverhältnissen miteinander vermischt.

Die erfindungsgemässen Wirkstoffkombinationen sind gegen eine Vielzahl Gram-positiver und Gram-negativer Bakterien wirksam, wie z.B. Escherichia coli, Proteus mirabilis, Pseudomonas aeruginosa, Staphylococcus aureus, Streptococcus pyogenes, Streptococcus faecalis, Streptococcus agalactiae, Streptococcus dysgalactiae und Klebsiella aerogenes. Die Wirkstoffkombinationen können dementsprechend zur Behandlung und Prophylaxe bakterieller Infektionen verwendet werden. Sie können oral oder parenteral verabreicht werden.

Die in vitro-Akivität der erfindungsgemässen Wirkstoffkombinationen wurde folgendermassen nachgewiesen:

Es wurden konzentrierte Lösungen von Gemischen der Verbindungen der Formel I und dem Antibiotikum in den gewünschten Gewichtsverhältnissen hergestellt, die dann beliebig verdünnt wurden. Aliquote Teile der verdünnten Lösungen wurden mit einem geeigneten Nähragar in Petrischalen gemischt. Zum Vergleich wurden ähnliche Agar-Schalen hergestellt, die ausser dem Nährmedium die Verbindung I bzw. das Antibiotikum alleine enthielten. Nach Oberflächeninokulation mit den Mikroorganismen wurde 24 Stunden bei 37°C aufbewahrt und dann die minimale Hemmkonzentration (M.I.C.) bestimmt und die F.I.C.-Indexe berechnet. Die Ergebnisse unter Verwendung repräsentativer Verbindungen der Formel I, nämlich (1R)-1-(N-Sarcosyl-L-norvalyl-L-norvalylamino)-ethylphosphonsäure, und (1R)-1-(N-Methyl-L-leucyl-L-norvalyl-L-norvalylamino)-ethylphosphonsäure und repräsentativer Antibiotika, nämlich Cephalexin und D-Cycloserin, sind in den folgenden Tabellen I-III zusammengestellt.

Tabelle I

Aktivität von Gemischen aus Cephalexin und (1R)-1-(N-Sarcosyl-L-norvalyl-L-norvalylamino)-ethylphosphonsäure gegen Proteus mirabilis, Staphylococcus aureus, Streptococcus faecalis und Streptococcus pyogenes

| Mikroorganismus | M.I.C. (µg/ml) | | | Peptid: Antibiotikum | F.I.C. Index |
|---|---|---|---|---|---|
| | Peptid | Antibiotikum | Gemisch | | |
| P. mirabilis | >128 | 22.6 | 1.3+2.7 | 1:2 | <0.13 |
| S. aureus | >128 | 45.2 | 5.3+10.7 | 1:2 | <0.28 |
| S. faecalis | 90.5 | 128 | 5.3+10.7 | 1:2 | 0.14 |
| S. pyogenes | 90.5 | 11.3 | 0.17+0.33 | 1:2 | 0.03 |

Tabelle II

Aktivität von Gemischen aus Cephalexin und (1R)-1-(N-Methyl-L-leucyl-L-norvalyl-L-norvalylamino)-ethylphosphonsäure gegen Staphylococcus aureus, Streptococcus pyogenes und Streptococcus faecalis

| Mikroorganismus | M.I.C. (µg/ml) | | | Peptid: Antibiotikum | F.I.C. Index |
|---|---|---|---|---|---|
| | Peptid | Antibiotikum | Gemisch | | |
| S. aureus | >64 | 64 | 5.3+10.7 | 1:2 | <0.25 |
| S. pyogenes | >64 | 8 | 0.33+0.66 | 1:2 | <0.088 |
| S. faecalis | 2 | 128 | 0.7+1.3 | 1:2 | 0.36 |

Tabelle III

Aktivität von Gemischen aus D-Cycloserin und (1R)-1-(N-Sarcosyl-L-norvalyl-L-norvalylamino)-ethylphosphonsäure gegen Escherichia coli, Staphylococcus aureus, Streptococcus agalactiae und Streptococcus dysgalactiae

| Mikro-organismus | M.I.C. (µg/ml) | | | Peptid: Antibiotikum | F.I.C. Index |
|---|---|---|---|---|---|
| | Peptid | Antibiotikum | Gemisch | | |
| E. coli | 2 | 32 | 1 | 1:1 | 0.27 |
| S. aureus | >128 | 16 | 2 | 1:1 | <0.07 |
| S.agalactiae | 45.2 | 90.5 | 2 | 1:1 | 0.03 |
| S.dysgalactiae | 2.8 | 128 | 2 | 1:1 | 0.36 |

Die in vivo-Aktivität der erfindungsgemässen Kombinationen wurde folgendermassen nachgewiesen:

Mäuse wurden intraperitoneal mit der 5-10fachen $LD_{99}$ eines pathogenen Organismus infiziert. In bestimmten Zeitintervallen nach der Infektion wurden jeweils Gruppen von Mäusen subcutan mit bestimmten Dosen des Antibiotikums, des Peptids und einer Anzahl Gemischen davon behandelt. Es wurde die Anzahl der Mäuse bestimmt, die für jede Behandlung 7 Tage nach der Infektion noch am Leben waren und daraus die $CD_{50}$ errechnet. Die F.I.C.-Indexe wurden für die Gemische in der üblichen Weise berechnet. Die Ergebnisse, die mit (1R)-1-(N-Sarcosyl-L-norvalyl-L-norvalylamino)-ethylphosphonsäure als einem Peptid der Formel I und Cephalexin bzw. D-Cycloserin als Antibiotika erhalten wurden, sind in den folgenden Tabellen IV und V zusammengestellt.

Tabelle IV

Chemotherapeutische Aktivität ($CD_{50}$ mg/kg, s.c.) von Gemischen aus Cephalexin und (1R)-1-(N-Sarcosyl-L-norvalyl-L-norvalylamino)-ethylphosphonsäure bei bakteriellen Infektionen an Mäusen

| Infektions-organismus | $CD_{50}$ | | | Peptid: Antibio-tikum | F.I.C.Index |
|---|---|---|---|---|---|
| | Peptid | Antibio-tikum | Gemisch | | |
| K.aerogenes | 18.3 | 9.3 | 2.3 + 2.3 | 1:1 | 0.38 |
| S.faecalis | 1.9 | >200 | <0.35 +<0.35 | 1:1 | <0.18 |
| Ps aerugin-osa | >200 | >200 | 46.6 + 46.6 | 1:1 | <0.46 |
| P.mirabilis | >200 | 20.3 | 9.3 + 9.3 | 1:1 | <0.50 |
| S.pyogenes | 70.7 | 2.7 | 1.3 + 1.3 | 1:1 | 0.50 |
| E.coli | 1.4 | 5.3 | 0.5 + 0.5 | 1:1 | 0.42 |
| S.aureus | >200 | 32.5 | 5.6 + 5.6 | 1:1 | <0.20 |

Tabelle V

Chemotherapeutische Aktivität ($CD_{50}$ mg/kg, s.c.) von Gemischen aus D-Cycloserin und (1R)-1-(N-Sarcosyl-L-norvalyl-L-norvalylamino)-ethylphosphonsäure bei bakteriellen Infektionen an Mäusen

| Infektions-organismus | $CD_{50}$ [mg/kg] s.c. | | | Peptid: Antibiotikum | F.I.C. Index |
|---|---|---|---|---|---|
| | Peptid | Antibio-tikum | Gemisch | | |
| E. coli | 1.1 | 28 | 0.4+ 0.4 | 1:1 | 0.39 |
| K. aerogenes | >30 | 42 | 4.1+ 4.1 | 1:1 | <0.23 |
| P. mirabilis | >200 | >200 | 31 +31 | 1:1 | <0.31 |
| Ps. aeruginosa | >300 | 61 | <7.5+<7.5 | 1:1 | <0.15 |
| S. faecalis | 1.8 | 123 | 0.6+ 0.6 | 1:1 | 0.34 |
| S. pyogenes | 67 | >200 | 19 +19 | 1:1 | <0.38 |
| S. agalactiae | 194 | >300 | 27 +27 | 1:1 | <0.23 |
| S. dysgalactiae | 194 | 141 | 12 +12 | 1:1 | 0.15 |

Die erfindungsgemässen Wirkstoffkombinationen können in Form pharmazeutischer Präparate verabreicht werden, die ebenfalls Gegenstand der vorliegenden Erfindung sind. Es kommen die üblichen mit den Verbindungen der Formel I bzw. ihren Salzen und den Antibiotika verträglichen pharmazeutischen Träger für die Herstellung der Präparate in Frage. Träger für die enterale (z.B. orale) oder parenterale Applikation können flüssig oder fest, organischen oder anorganischen Charakters sein und umfassen beispielsweise Wasser, Gelatine, Mannit, mineralische Oele, vegetabile Oele, Gummi arabicum, Propylenglykole oder Polyalkylenglykole.

Die Herstellung der pharmazeutischen Präparate kann in an sich bekannter Weise geschehen, dadurch z.B. dass man die einzelnen Komponenten mit dem geeigneten Trägermaterial vermischt und in eine geeignete galenische Form bringt.

Die pharmazeutischen Präparate können in fester Form (z.B. als Lyophilisate) oder in flüssiger Form (z.B. als Lösungen, Suspensionen oder Emulsionen) vorliegen. Gegebenenfalls sind sie sterilisiert und bzw. oder enthalten weitere Hilfsstoffe wie Konservierungsmittel, Stabilisierungs-, Netz- oder Emulgiermittel, Mittel zur geschmacklichen Verbesserung, Salze zur Veränderung des osmotischen Druckes oder Puffersubstanzen. Bei Verwendung von Puffern kann der pH-Wert der Präparate innerhalb der üblichen Grenzen variieren.

Der Gehalt an Verbindungen der Formel I oder deren Salzen und dem Antibiotikum in den erfindungsgemässen pharmazeutischen Präparaten und deren Dosierung können innerhalb weiter Grenzen im Rahmen des üblichen variieren. Die optimalen Verhältnisse hängen ab von den tatsächlich verwendeten Komponenten, dem Applikationsweg, der Art der Infektion usw. So liegt beispielsweise eine geeignete tägliche Dosis bei parenteraler Applikation bei etwa 200-2000 mg des Gemisches der aktiven Komponenten, bei oraler Applikation bei 750-1500 mg. Diese Dosis kann als einmalige Dosis oder in Teildosen verabreicht werden und kann in besonderen Situationen je nach Verschreibung des Arztes aufgrund individueller Erfordernisse erhöht oder erniedrigt werden.

Die folgenden Beispiele erläutern die Erfindung:

## Beispiel 1

Hartgelatine-Kapseln enthaltend die folgenden Komponenten:

| | pro Kapsel |
|---|---|
| (1R)-1-(N-Sarcosyl-L-norvalyl-L-norvalylamino)-ethylphosphonsäure | 250,0 mg |
| Cephalexin | 25,0 mg |
| Maisstärke | 21,0 mg |
| Dioctylnatriumsulfosuccinat | 0,5 mg |
| Stearinsäure | 3,5 mg |
| Gesamtgewicht | 300,0 mg |

Die (1R)-1-(N-Sarcosyl-L-norvalyl-L-norvalylamino)-ethylphosphonsäure wurde mit 10%iger Maisstärke-Paste, die das Dioctylnatriumsulfosuccinat enthielt, granuliert. Das feuchte Granulat wurde getrocknet, gesiebt, sowie mit Cephalexin und Stearinsäure (vorgängig gesiebt) vermischt. Das erhaltene Gemisch wurde in Hartgelatine-Kapseln abgefüllt.

## Beispiel 2

Hartgelatine-Kapseln enthaltend die folgenden Komponenten:

| | pro Kapsel |
|---|---|
| (1R)-1-(N-Sarcosyl-L-norvalyl-L-norvalylamino)-ethylphosphonsäure | 125,0 mg |
| Cephalexin | 125,0 mg |
| Maisstärke | 20,0 mg |
| Mikrokristalline Cellulose | 34,0 mg |
| Dioctylnatriumsulfosuccinat | 0,5 mg |
| Stearinsäure | 5,5 mg |
| Gesamtgewicht | 310,0 mg |

Die Herstellung der Kapseln wurde in Analogie zu der

in Beispiel 1 beschriebenen Weise durchgeführt, wobei allerdings die mikrokristalline Cellulose vor Abfüllung in die Kapseln mit den anderen Bestandteilen vermischt wurde.

Beispiel 3

Pulvriges Gemisch zur Herstellung von Injektionslösungen, enthaltend die folgenden Bestandteile:

| | |
|---|---|
| Mecillinam | 100,0 mg |
| (1R)-1-(N-Sarcosyl-L-norvalyl-L-norvalylamino)-ethylphosphonsäure | 100,0 mg |
| Natriumcitrat | 40,0 mg |

Die Bestandteile wurden einzeln gemahlen und sorgfältig miteinander vermischt. Das Gemisch wurde unter sterilen Bedingungen in geeignete Behälter abgefüllt. Zur Herstellung einer Injektionslösung wird das erhaltene Gemisch in 2 ml Wasser für Injektionszwecke gelöst.

Beispiel 4

Hartgelatine-Kapseln, enthaltend die folgenden Bestandteile:

| | pro Kapsel |
|---|---|
| (1R)-1-(N-Sarcosyl-L-norvalyl-L-norvalylamino)-ethylphosphonsäure | 100,0 mg |
| D-Cycloserin | 100,0 mg |
| Magnesiumoxid | 35,0 mg |
| Polyvinylpyrrolidon | 5,0 mg |
| Magnesiumstearat | 10,0 mg |
| | 250,0 mg |

Die (1R)-1-(N-Sarcosyl-L-norvalyl-L-norvalylamino)-ethylphosphonsäure wird mit Polyvinylpyrrolidon, Magnesiumoxid und Wasser granuliert. Das feuchte Granulat wird getrocknet, gesiebt, mit D-Cycloserin und Magnesiumstearat

(vorgängig gesiebt) gemischt und in Hartgelatinekapseln abgefüllt.

## Beispiel 5

Es wird eine Injektionslösung hergestellt durch Auflösung von 200 mg gefriergetrocknetem D-Cycloserin und 200 ml gefriergetrockneter (1R)-1-(N-Sarcosyl-L-norvalyl-L-norvalylamino)-ethylphosphonsäure in einer sterilen Pufferlösung (pH 6,7). Die erhaltene Lösung wird unter sterilen Bedingungen filtriert und aseptisch gefriergetrocknet. Vor der Anwendung wird das gefriergetrocknete Material mit 10 ml eines Puffers rekonstituiert. Ein geeigneter Puffer kann folgendermassen zusammengesetzt sein:

| | |
|---|---|
| Natriumchlorid | 8,3 mg |
| Eisessig | 1,0 mg |
| Natriumhydroxid q.s. ad pH 4,5 | |
| Wasser für Injektionszwecke ad | 1,00 ml |

Patentansprüche

1. Wirkstoffkombinationen mit antibiotischen Eigenschaften, dadurch gekennzeichnet, dass sie ein Peptidderivat der allgemeinen Formel

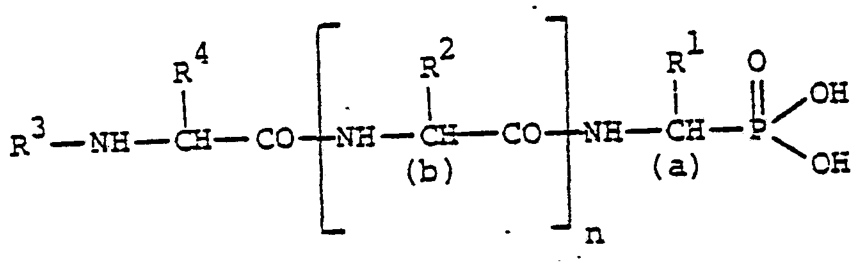

(I)

worin $R^1$ Wasserstoff, Methyl, Hydroxymethyl, eine Mono-, Di- oder Trihalogenmethylgruppe ist;
$R^2$ einen für eine normalerweise in Proteinen vorkommende α-Aminosäure charakteristischen Rest oder einen Niederalkyl- oder Hydroxy-niederalkylrest, der charakterisch ist für eine in Proteinen normalerweise nicht vorkommende α-Aminosäure, darstellt;
$R^3$ Niederalkyl, Nieder-cycloalkyl, Nieder-alkenyl, Aryl oder Aryl-niederalkyl ist;
$R^4$ Wasserstoff oder Niederalkyl bedeutet;
n 2 oder 3 bedeutet und
die Konfigurationen an den C-Atomen (a) und (b) R (wenn $R^1 \neq H$) bzw. L sind,
oder ein physiologisch verträgliches Salz einer solchen Verbindung und ein Antibiotikum enthalten.

2. Wirkstoffkombinationen gemäss Anspruch 1, dadurch gekennzeichnet, dass in dem Peptidderivat der Formel I $R^1$ Wasserstoff oder Methyl ist.

3. Wirkstoffkombinationen gemäss Anspruch 1 oder 2, dadurch gekennzeichnet, dass in dem Peptidderivat der Formel I $R^2$ ein für eine normalerweise in Proteinen vor-

kommende α-Aminosäure charakteristischer Rest oder ein Niederalkylrest ist, der charakteristisch ist für eine normalerweise in Proteinen nicht vorkommende α-Aminosäure.

4. Wirkstoffkombinationen gemäss einem der Ansprüche 1-3, dadurch gekennzeichnet, dass in dem Peptidderivat der Formel I $R^3$ Niederalkyl ist.

5. Wirkstoffkombinationen gemäss einem der Ansprüche 1-4, dadurch gekennzeichnet, dass das Peptidderivat der Formel I (1R)-1-(N-Sarkosyl-L-norvalylamino)-ethylphosphonsäure ist.

6. Wirkstoffkombinationen gemäss einem der Ansprüche 1-5, dadurch gekennzeichnet, dass das Antibiotikum ein β-Lactam-Antibiotikum ist.

7. Wirkstoffkombinationen gemäss einem der Ansprüche 1-5, dadurch gekennzeichnet, dass das Antibiotikum ein Penicillin, ein Cephalosporin oder ein monocyclisches β-Lactam-Antibiotikum ist.

8. Wirkstoffkombinationen gemäss Anspruch 7, dadurch gekennzeichnet, dass es sich bei dem Penicillin um Ampicillin, Carbenicillin, Penicillin G, Sulbenicillin, Mecillinam, Pivmecillinam, Phenethicillin, Methicillin, Propicillin, Ticarcillin, Amoxycillin oder Piperacillin handelt.

9. Wirkstoffkombinationen gemäss Anspruch 7, dadurch gekennzeichnet, dass es sich bei dem Cephalosporin um Cephalexin, Cephazolin, Cefoxitin, Cephradin, Cefsulodin, Cephamandol, Cephaloridin, Cephaloglycin, Cefatrizin, Cefacetril, Cefuroxim, Cefactor, Cefotaxim oder Cefazedon handelt.

10. Wirkstoffkombinationen gemäss einem der Ansprüche

1-5, dadurch gekennzeichnet, dass das Antibiotikum D-Cycloserin, Rifampicin, Phosphonomycin, Gentamycin, Vancomycin oder Kanamycin ist.

11. Wirkstoffkombinationen gemäss einem der Ansprüche 1-10 zur Therapie und Prophylaxe bakterieller Infektionen.

12. Verfahren zur Herstellung von Wirkstoffkombinationen gemäss einem der Ansprüche 1-10, dadurch gekennzeichnet, dass man eine Verbindung der Formel I oder ein physiologisch verträgliches Salz einer solchen Verbindung und ein Antibiotikum miteinander vermischt.

13. Pharmazeutische Präparate auf der Basis einer Wirkstoffkombination gemäss einem der Ansprüche 1-10.

14. Verwendung einer Wirkstoffkombination gemäss einem der Ansprüche 1-10 als antibakterielles Mittel.

***

0026409

Europäisches Patentamt

# EUROPÄISCHER RECHERCHENBERICHT

Nummer der Anmeldung

EP 80 10 5635

## EINSCHLÄGIGE DOKUMENTE

| Kategorie | Kennzeichnung des Dokuments mit Angabe, soweit erforderlich, der maßgeblichen Teile | betrifft Anspruch | KLASSIFIKATION DER ANMELDUNG (Int. Cl.3) |
|---|---|---|---|
| | US - A - 4 134 972 (F.R. ATHERTON)<br>* Spalte 1 und Spalte 2, Zeilen 1,2 *<br>---- | 1-3,6-11 | A 61 K 37/16//<br>(A 61 K 37/16, 31/42)<br>(A 61 K 37/16, 31/43)<br>(A 61 K 37/16, 31/545) |

RECHERCHIERTE SACHGEBIETE (Int. Cl.3)

A 61 K 37/16
31/545
31/43
31/42
C 07 F 9/00
C 07 D 499/00
501/00
C 07 C 103/00

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

Der vorliegende Recherchenbericht wurde für alle Patentansprüche erstellt.

| Recherchenort | Abschlußdatum der Recherche | Prüfer |
|---|---|---|
| Den Haag | 11-12-1980 | CHOULY |